# EUROPEAN PATENT SPECIFICATION

(11) **EP 4 681 685 B1**
(45) Date of publication and mention of the grant of the patent: **09.12.2026**
(21) Application number: 25189691.6
(22) Date of filing: 15.07.2025
(51) Int. Cl.: A61F 2/58

(54) **A PROSTHETIC HAND**
PROTHETISCHE HAND
PROTHÈSE DE MAIN

(30) Priority: 17.07.2024 GB 202410428
(43) Date of publication of application: 21.01.2026
(73) Proprietor: OPEN BIONICS LTD., Bristol BS1 2NB (GB)
(72) Inventor: WOOD, Steve, Bristol BS1 2NB (GB); WARREN, Leigh, Bristol BS1 2NB (GB)
(74) Representative: Haseltine Lake Kempner LLP

(56) References cited:
- GB-A- 2 528 049
- GB-A- 2 577 500
- US-B2- 11 351 042

## Description

### FIELD OF THE INVENTION

The present disclosure relates to a prosthetic hand and particularly, although not exclusively, relates to a prosthetic hand comprising a proximal thumb part and a distal thumb part, wherein the proximal thumb part is coupled to a palm part to form a first joint with a first axis of rotation, and the distal thumb part is coupled to the proximal thumb part to form a second joint with a second axis of rotation.

### BACKGROUND OF THE INVENTION

Prosthetic thumbs of currently existing prosthetic hands suffer from a limited range of motion. The user is typically unable to form a desired position with the prosthetic hand. For example, a user may be unable to abduct and/or flex the prosthetic thumb to form a fist, similarly they may be unable to extend and/or adduct the prosthetic thumb to pinch or grab an object. It is therefore desirable to provide a prosthetic hand comprising a prosthetic thumb, wherein the prosthetic thumb is rotatable between a plurality of functional positions.

To rotate a prosthetic thumb about two different axes of rotation, prosthetic hands tend to use dual motor actuation systems. Disposing multiple motors in the prosthetic hand increases its weight and bulk. Accordingly, many designs dispose at least one of the dual-motors adjacent to the user's wrist. This, however, results in electrical cables being threaded through the prosthetic hand to the prosthetic thumb, which compromises a watertightness of the design. Accordingly, it is desirable to provide a prosthetic hand with a comparatively reduced bulk and weight, and an improved robustness to water ingress.

GB 2 528 049 discloses a mechanical hand comprising: a plurality of digit assemblies selectively moveable by a respective drive assembly about a pivot axis along a flexion/extension plane and between an open position and a closed position; and a controller operatively coupled to each of the drive assemblies; wherein the controller is configured to move at least one of the digit assemblies to form or maintain a desired grip responsive to at least one sensed parameter associated with at least one of the digit assemblies.

### SUMMARY OF THE INVENTION

According to a specific aspect, there is provided a prosthetic hand comprising:
a palm part;
a proximal thumb part coupled to the palm part to form a first joint with a first axis of rotation, wherein the proximal thumb part is configured to rotate about the first axis of rotation;
a distal thumb part coupled to the proximal thumb part to form a second joint with a second axis of rotation, wherein the distal thumb part is configured to rotate about the second axis of rotation;
a rotary actuator configured to rotate the proximal thumb part and distal thumb part about the first axis of rotation and the second axis of rotation respectively; and
a locking mechanism having a first configuration and a second configuration, the locking mechanism being configured such that:
   in the first configuration, the distal thumb part is rotatably locked (e.g. resiliently) relative to the proximal thumb part about the second axis of rotation such that the rotary actuator rotates both the distal thumb part and proximal thumb part about the first axis of rotation, and
   in the second configuration, the proximal thumb part is rotatably locked (e.g. resiliently) relative to the palm part about the first axis of rotation such that the rotary actuator rotates the distal thumb part relative to the proximal thumb part about the second axis of rotation.

The proximal thumb part may rotate between a first limit of travel and a second limit of travel. The locking mechanism may be configured to switch between the first and second configurations at at least one of the first and second limits of travel.

The locking mechanism may comprise one or more movable locking element(s). The movable locking element(s) may be configured to selectively restrict (e.g. prevent) relative movement of the palm part and proximal thumb part. The movable locking element(s) may be configured to selectively restrict (e.g. prevent) relative movement of the distal thumb part and proximal thumb part.

The locking mechanism may comprise a movable locking element. The movable locking element may be configured to restrict (e.g. prevent) relative movement of the palm part and proximal thumb part when in a first position. The movable locking element may be configured to restrict (e.g. prevent) relative movement of the distal thumb part and proximal thumb part when in a second position.

The locking mechanism may be configured such that the locking element is urged into the first position by movement of the distal thumb part when the proximal thumb part is at the first and/or second limits of travel.

The locking mechanism may comprise a biasing element. The biasing element may be configured to bias the locking element into the second position.

The biasing element and the locking element may be magnetically attracted to one another.

The locking mechanism may comprise a first movable locking element configured to restrict relative movement of the palm part and proximal thumb part when the first movable locking element is in an extended position. The locking mechanism may comprise a second movable locking element configured to restrict relative movement of the distal thumb part and proximal thumb part when the second movable locking element is in an extended position. The first movable locking element may be part of a spring-loaded plunger and/or the second movable locking element may be part of a spring-loaded plunger.

The proximal thumb part may comprise a first shaft configured to rotate about the first axis of rotation. The first shaft may be positioned such that the first axis of rotation passes through the first shaft.

A longitudinal axis of the first shaft may be substantially parallel and coincidental to the first axis of rotation.

The first shaft may comprise a recess. The locking mechanism may comprise a locking element (e.g. second movable locking element) configured to engage the recess. The first shaft may not be rotatable relative to a surrounding casing of the proximal thumb part when the locking element engages the recess. The first shaft may be rotatable relative to the surrounding casing of the proximal thumb part when the locking element does not engage the recess.

The locking element may comprise an at least partially magnetic object. The first shaft may comprise a magnet configured to attract the at least partially magnetic object into the recess.

The locking element may comprise a spring-loaded plunger.

The palm part may comprise the rotary actuator. The rotary actuator may be configured to drive the first shaft through a gear mechanism.

The gear mechanism may comprise a worm screw and a worm wheel. The worm screw may be coupled to the rotary actuator and to the worm wheel. The worm wheel may be at least partially disposed around the first shaft.

The distal thumb part may comprise a second shaft configured to rotate about the second axis of rotation. The second shaft may be positioned such that the second axis of rotation passes through the second shaft.

A longitudinal axis of the second shaft may be substantially parallel and coincidental to the second axis of rotation.

The distal thumb part may comprise a third shaft configured to rotate about the second axis of rotation. A longitudinal axis of the third shaft may be substantially parallel and offset to the second axis of rotation. An arm or linkage may couple the first shaft to the third shaft. The arm may comprise at least one link.

The arm may comprise a first link, a second link, and a third link. The first link and the second link may be coupled to one another. The second link and the third link may be coupled to one another. The first link may be coupled to the first shaft. The third link may be coupled to the third shaft.

The second axis of rotation may be substantially perpendicular to the first axis of rotation.

The distal thumb part may comprise a biasing spring. The biasing spring may be configured to return the distal thumb part to a neutral position.

The proximal thumb part and the distal thumb part may be configured to move sequentially through a plurality of positions. The rotary actuator may be configured to move the proximal thumb part and the distal thumb part through the sequence.

These and other aspects will be apparent from and elucidated with reference to the embodiment(s) described hereinafter. Also, features described with respect to one aspect or embodiment may also be applied to other aspects or embodiments.

### BRIEF DESCRIPTION OF THE DRAWINGS

Exemplary embodiments will now be described, by way of example only, with reference to the following drawings, in which:
Figures 1a and 1b (collectively Figure 1) are perspective views of a prosthetic hand according to an example of the present disclosure, and show a thumb part of the prosthetic hand in an open reposition position and an open opposition position respectively;
Figures 2a and 2b (collectively Figure 2) are perspective views of the thumb part and a rotary actuator of the prosthetic hand according to an example of the present disclosure, and show the thumb part in an open position and a closed position respectively;
Figure 3 is a sectional view of the prosthetic hand according to an example of the present disclosure;
Figure 4 is a sectional view of the thumb part and rotary actuator of the prosthetic hand according to an example of the present disclosure;
Figure 5 is a side view of an arm coupling a shaft of a proximal thumb part to a distal thumb part according to an example of the present disclosure;
Figures 6a, 6b, 6c and 6d (collectively Figure 6) are sectional side views of the thumb part and rotary actuator of the prosthetic hand according to an example of the present disclosure, and show a locking mechanism of the prosthetic hand in a closed opposition position, an open opposition position, an open reposition position, and a closed reposition position respectively;
Figure 7 is a perspective view of a coupling mount part of the proximal thumb part according to an example of the present disclosure;
Figures 8a and 8b (collectively Figure 8) are sectional side views of the thumb part of the prosthetic hand according to an example of the present disclosure, and show the arm coupling the proximal thumb part to the distal thumb part in an open and closed position respectively;
Figure 9 is a side sectional view of the proximal thumb part and a palm part of the prosthetic hand according to an example of the present disclosure;
Figure 10 is a side sectional view of a proximal thumb part and a palm part of the prosthetic hand according to a further example of the present disclosure;
Figures 11a and 11b (collectively Figure 11) are sectional views of the of the thumb part of the prosthetic hand according to the further example of the present disclosure and show the thumb part of the prosthetic hand in an open reposition position and an open opposition position respectively;
Figures 12a and 12b (collectively Figure 12) are additional sectional views of the thumb part of the prosthetic hand according to the further example of the present disclosure and show the thumb part of the prosthetic hand in an open reposition position and a closed reposition position respectively;
Figure 13 is a further sectional view of the thumb part of the prosthetic hand according to the further example of the present disclosure and shows the thumb part of the prosthetic hand in an open reposition position; and
Figure 14 is a chart depicting the progression of the prosthetic hand according to the example and further example of the present disclosure between the different positions of the thumb part.

### DETAILED DESCRIPTION OF EMBODIMENTS

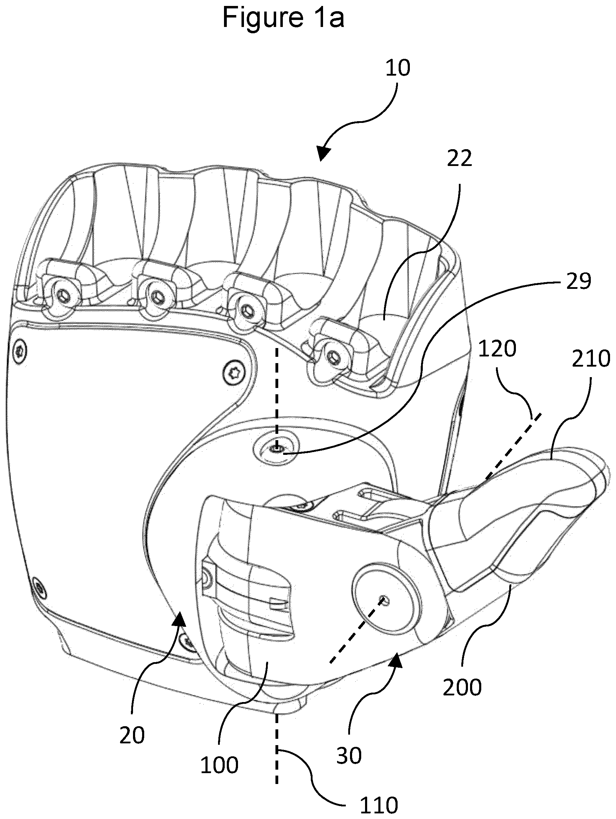

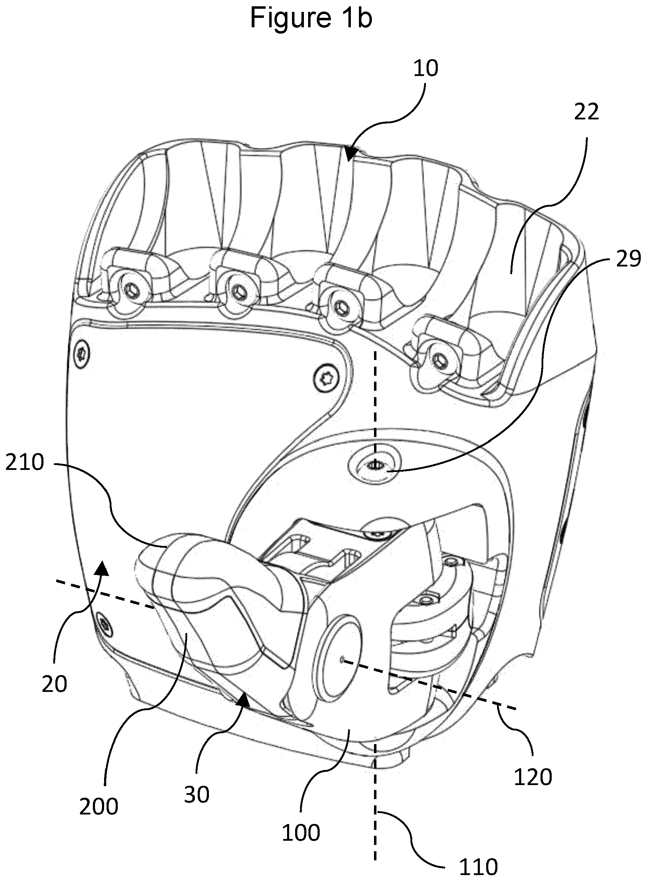

With reference to Figure 1, a prosthetic hand 10 according to an example of the present disclosure comprises a palm part 20 and a thumb part 30.

The thumb part 30 comprises a proximal thumb part 100 and a distal thumb part 200. The proximal thumb part 100 may be coupled to the palm part 20, e.g. at a first end of the proximal thumb part. The proximal thumb part 100 may be rotatably coupled to the palm part 20. In particular, the proximal thumb part 100 may be coupled to the palm part 20 to form a first joint with a first axis of rotation 110. The proximal thumb part 100 may be configured to rotate about the first axis of rotation 110. The proximal thumb part 100 may be configured to rotate about the first axis of rotation 110 relative to the palm part 20.

The distal thumb part 200 may be coupled to the proximal thumb part 100, e.g. at a second end of the proximal thumb part. The coupling between the distal thumb part 200 and proximal thumb part 100 may form a second joint with a second axis of rotation 120. The distal thumb part 200 may be configured to rotate about the second axis of rotation 120. The distal thumb part 200 may be configured to rotate about the second axis of rotation 120 relative to the proximal thumb part 100. As will be further elaborated below, in one configuration the distal thumb part 200 may be rotatably locked relative to the proximal thumb part 100 about the second axis of rotation 120. Accordingly, the distal thumb part 200 and proximal thumb part 100 may rotate together about the first axis of rotation 110. Additionally, in another configuration, the proximal thumb part 100 may be rotatably locked relative to the palm part 20 about the first axis of rotation 110 and the distal thumb part 200 may be rotated relative to the proximal thumb part 100 about the second axis of rotation 120.

As shown in Figures 1a and 1b respectively, the proximal thumb part 100 may be configured to rotate about the first axis of rotation 110 between a 'reposition position' and an 'opposition position'. The term 'reposition position' may refer to when the proximal thumb part 100 is at a maximum extension. As shown in Figure 1a, in the reposition position the proximal thumb part 100 may extend outwards and away from a side of the palm part 20. Additionally or alternatively, the term 'reposition position' may refer to when the second axis of rotation 120 is substantially parallel to a sagittal axis of the palm part 20. The term 'opposition position' may refer to when the proximal thumb part 100 is at a maximum abduction. As shown in Figure 1b, in the opposition position the proximal thumb part 100 may extend over a surface of the palm part 20. Additionally or alternatively, the term 'opposition position' may refer to when the second axis of rotation 120 is substantially parallel a transverse axis of the palm part 20. As further shown in Figure 1, the palm part 20 may comprise at least one slot 22. The at least one slot 22 may be configured to receive a prosthetic finger. Accordingly, in the opposition position, at least a part of the thumb part 30 (e.g. the distal thumb part 200) may be configured to face or oppose a prosthetic finger coupled to the palm part 20. For example, in the opposition position, a surface 210 of the distal thumb part 200 may be able to face or oppose a surface of a prosthetic index finger coupled to the palm part 20.

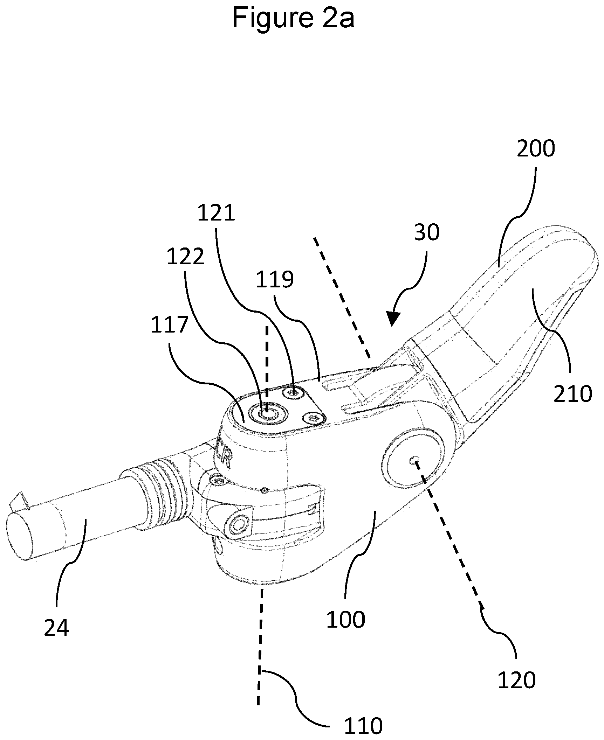

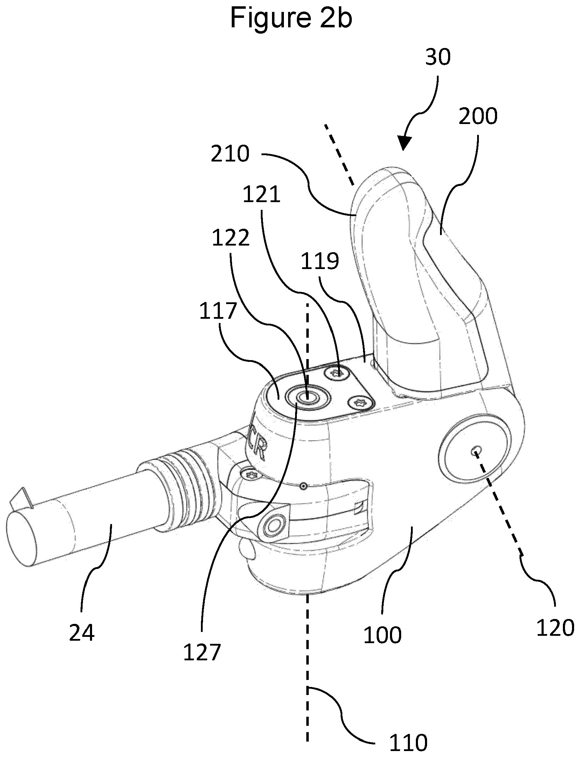

As shown in Figures 2a and 2b respectively, the distal thumb part 200 may be configured to rotate about the second axis of rotation 120 between an 'open position' and a 'closed position'. The 'open position' may refer to when the distal thumb part 200 extends or points away from the palm part 20 and/or the proximal thumb part 100. Additionally or alternatively, the 'open position' may refer to when a longitudinal axis of the distal thumb part 200 is rotated to an angle within a range of 0 to 90° clockwise about the second axis of rotation 120. The 'closed position' may refer to when the distal thumb part 200 extends or points towards the proximal thumb part 100 and/or the palm part 20. As best depicted in Figure 2b, in the closed position the surface 210 of the distal thumb part 200 may at least partially extend over or oppose a surface of the proximal thumb part 100 and/or palm part 20.

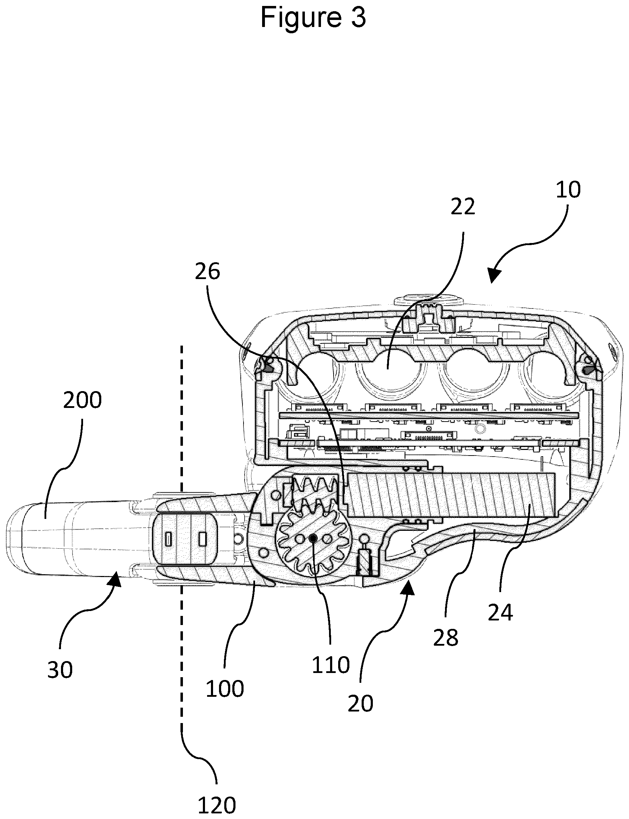

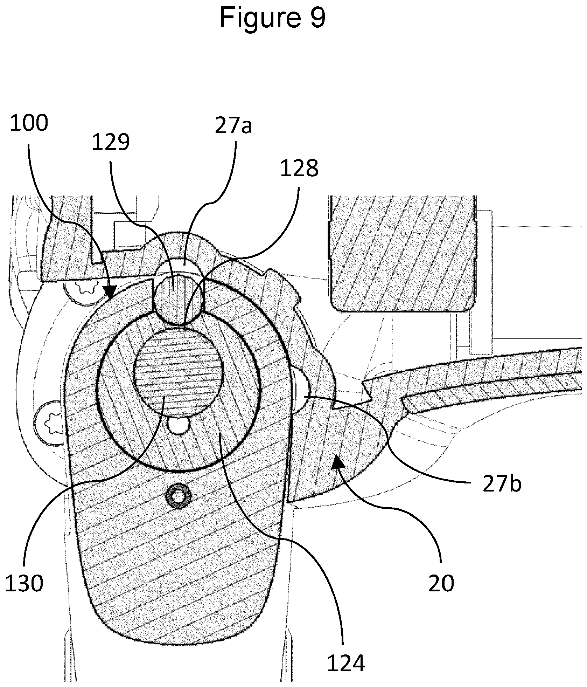

Accordingly, the proximal thumb part 100 may rotate about the first axis of rotation 110 between a first limit of travel (e.g. the reposition position) and a second limit of travel (e.g. the opposition position). As best shown in Figure 9, at the first limit of travel, a surface of the proximal thumb part 100 may abut a corresponding surface of the palm part 20, to thereby prevent the proximal thumb part 100 from over-rotating past the reposition position. Similarly, at the second limit of travel, a further surface of the proximal thumb part 100 may abut a corresponding further surface of the palm part 20, to thereby prevent the proximal thumb part 100 from over-rotating past the opposition position. The distal thumb part 200 may rotate about the second axis of rotation 120 between the open position and the closed position. As best shown in Figure 3, in order to cause such a rotation of the proximal thumb part 100 and/or the distal thumb part 200, the prosthetic hand 10 may further comprise an actuator, such as a rotary actuator 24. Although only a single actuator (rotary actuator 24) is shown, it is noted that the prosthetic hand 10 may comprise one or more actuators, e.g. with other actuators actuating fingers of the prosthetic hand. However, the prosthetic hand may comprise only a single actuator for actuating both the proximal and distal thumb parts. It is further noted that other actuator type(s), such as at least one linear actuator, may be used in addition to, or as an alternative to, the at least one rotary actuator 24. The rotary actuator 24 may comprise a motor, such as a stepper motor. The rotary actuator 24 may further comprise at least one sensor, such that a controller (not shown) may determine a position of the thumb part 30 in a movement sequence. For example, the actuator 24 may comprise at least one sensor that allows a controller to determine whether the proximal thumb part 100 is in the reposition position or the opposition position, and whether the distal thumb part 200 is in the open position or the closed position.

Referring still to Figure 3, the rotary actuator 24 may be disposed in the palm part 20. In other words, the palm part 20 may comprise the rotary actuator 24. The palm part 20 may at least partially enclose or house the rotary actuator 24. The rotary actuator 24 may extend along a transverse axis of the palm part 20. Particularly, a longitudinal axis of the rotary actuator 24 may be substantially parallel to a transverse axis of the palm part 20. The rotary actuator 24 may be disposed adjacent to the proximal thumb part 100. In particular, a coupling end 26 of the rotary actuator 24 may be disposed adjacent to the proximal thumb part 100. Additionally, the rotary actuator 24 may be disposed proximate to a bottom portion 28 of the palm part 20 (e.g. closest to a palm surface). The bottom portion 28 of the palm part 20 may be a bottom surface on the inside of the palm part 20. The longitudinal axis of the rotary actuator 24 may be perpendicular to the first axis of rotation 110. The longitudinal axis of the rotary actuator 24 may be spaced apart or offset from the first axis of rotation 110. In other words, the longitudinal axis of the rotary actuator 24 may not intersect with the first axis of rotation 110.

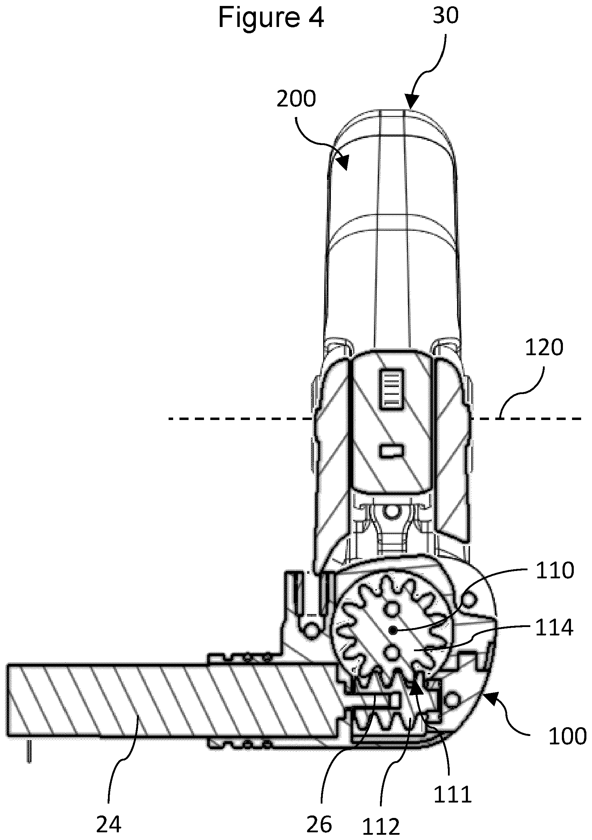

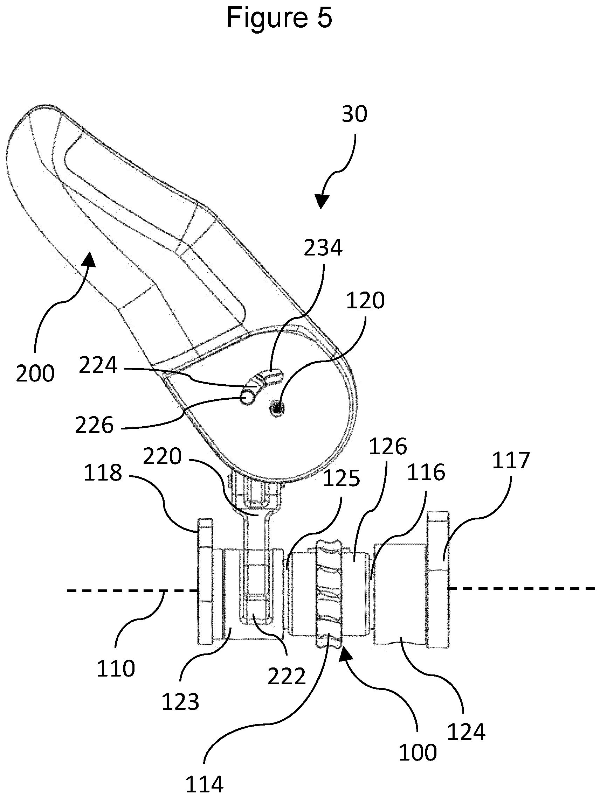

Referring now to Figure 4, the rotary actuator 24 may be coupled to the thumb part 30 by a gear mechanism 111. The proximal thumb part 100 may at least partially house, or may be at least partially disposed around, a housing for the gear mechanism 111. The coupling end 26 of the rotary actuator 24 may extend into the gear mechanism 111. In other words, the coupling end 26 of the rotary actuator 24 may couple the rotary actuator 24 to the gear mechanism 111. In the example shown in Figure 4, the gear mechanism 111 comprises a worm screw 112 and a worm wheel 114. It is noted, however, that the gear mechanism may comprise any other alternative gear types and/or arrangements. For example, the gear mechanism 111 may further comprise at least one idler gear. Additionally or alternatively, the gear mechanism 111 may comprise an arrangement of any type of gear(s), e.g. bevel gears, screw gears, spur gears, etc. The rotary actuator 24 may be coupled to the worm screw 112. The worm screw 112 may be coupled to the worm wheel 114. The worm screw 112 may be configured to engage the worm wheel 114. As best shown in Figure 5, the worm wheel 114 may be coupled to a first shaft 116 of the proximal thumb part 100. Specifically, the worm wheel 114 may be rigidly coupled to the shaft 116 of the proximal thumb part 100. The worm wheel 114 may be at least partially disposed around the shaft 116. Additionally or alternatively, a joining pin may extend through at least a portion of the worm wheel 114 and through at least a portion of the shaft 116, to rigidly couple the worm wheel 114 to the shaft 116 of the proximal thumb part 100. Accordingly, torque from the rotary actuator 24 may be transmitted or imparted to the shaft 116 of the proximal thumb part 100 via the worm screw 112 and the worm wheel 114. It is noted that the gear mechanism 111 may be considered an interface between the proximal thumb part 100 and the palm part 20. It is further noted that the longitudinal axis of the rotary actuator 24 may be substantially perpendicular to the longitudinal axis of the shaft 116.

Referring still to Figure 5, the shaft 116 of the proximal thumb part 100 may be rotatably received in the proximal thumb part 100. The shaft 116 may extend through the proximal thumb part 100. Specifically, a longitudinal axis of the shaft 116 may be substantially parallel to the first axis of rotation 110. In particular, the longitudinal axis of the shaft 116 may be substantially parallel and coincidental to the first axis of rotation 110. The shaft 116 may extend between a first end plate 117 and a second end plate 118. The shaft 116 may be rotatably coupled to the first end plate 117 and/or the second end plate 118 by any suitable fastening means, e.g. at least one joining pin, screw, rivet, etc. As shown in Figure 2, the first end plate 117 and/or the second end plate 118 may, in turn, be coupled to a casing 119 of the proximal thumb part 100. For example, first end plate 117 and/or second end plate 118 may be coupled to the casing 119 of the proximal thumb part 100 by at least one bolt or screw 121. Still referring to Figure 2, the first end plate 117 and/or the second end plate 118 may further comprise a recess or cavity 122. The recess of cavity 122 of the first/second end plate 117, 118 may be configured to receive a screw, bolt, and/or any other suitable fastener. The recess or cavity 122 of the first/second end plate 117, 118 may comprise a thread. Accordingly, and turning now to Figure 1, a rivet, bolt, screw, or any other fastener 29 may be inserted through the palm part 20 and be received by the corresponding recess or cavity 122 of the first/second end plate 117, 118, to thereby couple the palm part 20 to the proximal thumb part 30. Referring again to Figure 2, it is noted that a bearing 127, e.g. a rotary bearing, may be disposed in the recess or cavity 122 of end plate 117 and/or end plate 118. Specifically, the bearing 127 may be configured to at least partially surround the fastener 29. For example, where the fastener 29 is a screw, the bearing 127 may at least partially surround the thread of the screw. Or, where the fastener 29 is a rivet, the bearing 127 may at least partially surround the pin of the rivet.

Turning back to Figure 5, the shaft 116 of the proximal thumb part 100 may be unitary, i.e. it may be a single part. For example, it may be a single part that extends from end plate 117 to end plate 118. The shaft 116 may be considered a single unitary part comprising three distinct portions. Alternatively, and as depicted in Figure 5, the shaft 116 may comprise three distinct parts. For example, the shaft 116 may comprise three separate parts rigidly coupled to one another. The shaft parts may be coupled to one another using any suitable means, e.g. joining pins, dowels, adhesive, or any other suitable fasteners. The portions or parts of the shaft 116 may comprise a coupling mount 123, a lock portion 124, and a centre connector 125. The coupling mount 123, lock portion 124, and centre connector 125 may each be of a substantially similar shape and size to one another. For example, they may be cylindrical and comprise a substantially similar diameter to one another. Alternatively, they may be of different shapes and sizes to one another. For example, as shown in Figure 5, the coupling mount 123, centre connector 125, and lock portion 124 may be of different diameters to one another.

The coupling mount 123 may be disposed proximate to the second end plate 118. The lock portion 124 may be disposed proximate to the first end plate 117. The centre connector 125 may be disposed between the coupling mount 123 and the lock portion 124. At least one sleeve, bushing, bearing, etc. may be disposed around the centre connector 125. For example, at least one bushing 126 may be disposed between the worm wheel 114 and the centre connector 125. The at least one bushing 126 may couple the worm wheel 114 to the centre connector 125. Therefore, torque from the rotary actuator 24 may be transmitted or imparted through the worm wheel 114 and to the centre connector 125 of the shaft 116.

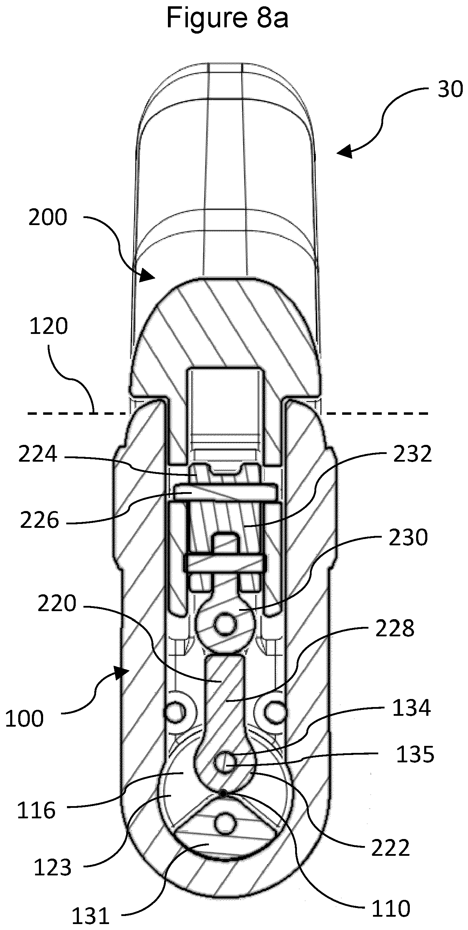

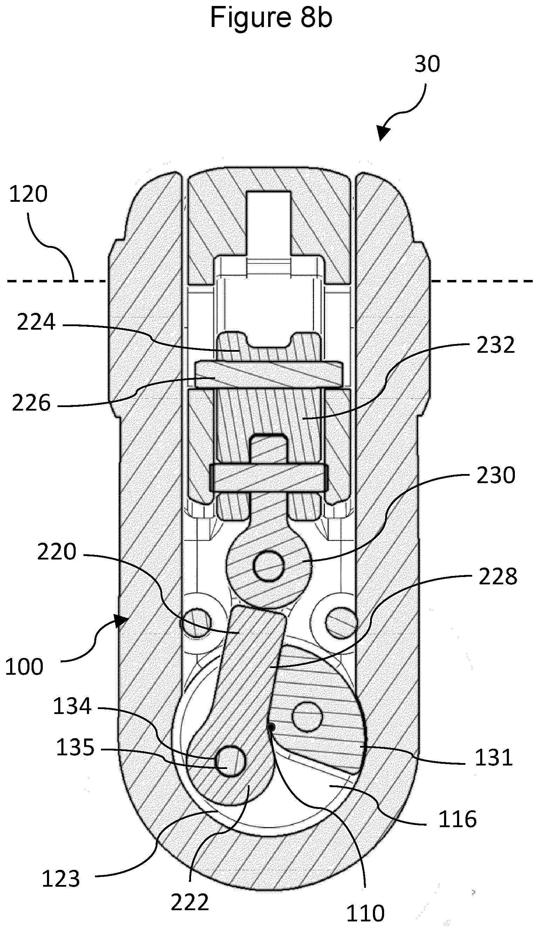

The coupling mount 123 may be coupled to an arm 220. A first end 222 of the arm 220 may be coupled to the coupling mount 123 of the shaft 116, and a second end 224 of the arm 220 may be coupled to the distal thumb part 200. Accordingly, the arm 220 may couple the proximal thumb part 100 to the distal thumb part 200. Specifically, the arm 220 may couple the shaft 116 of the proximal thumb part 100 to a pin or dowel 226 that extends through the distal thumb part 200. More specifically, the arm 220 may couple the coupling mount 123 of the proximal thumb part 100 to the pin or dowel 226 that extends through the distal thumb part 200. The coupling mount 123 may be rigidly coupled to the centre connector 125. Alternatively, the coupling mount 123 and the centre connector 125 may be unitary. Therefore, torque transferred from the rotary actuator 24 to the centre connector 125 via the gear mechanism 111, may in turn be transmitted to the coupling mount 123. The coupling mount 123 may therefore rotate about the first axis of rotation 110, which, in turn, may cause the arm 220 to exert a force on the distal thumb part 200. As will be further discussed below (in relation to Figure 8), such a force may cause the distal thumb part to rotate about the second axis of rotation 120, e.g. between the open and closed positions.

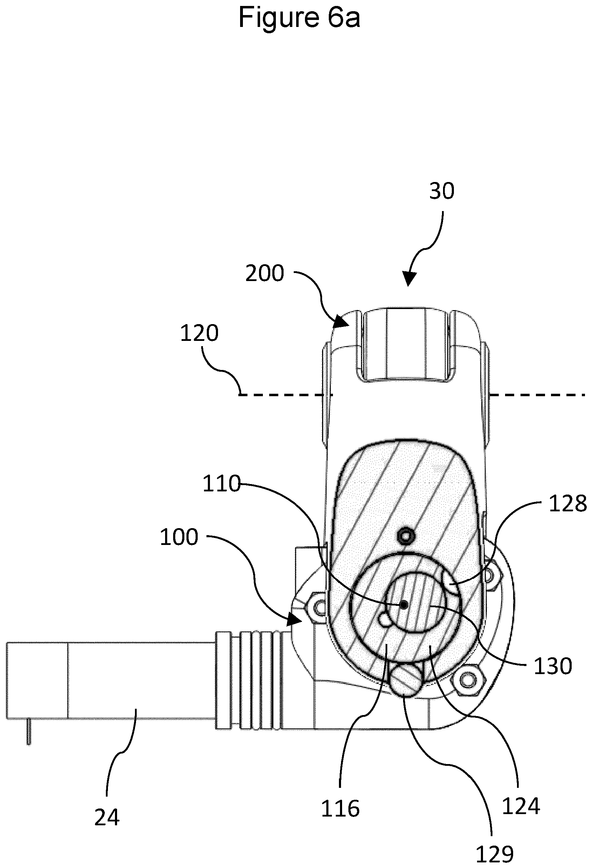

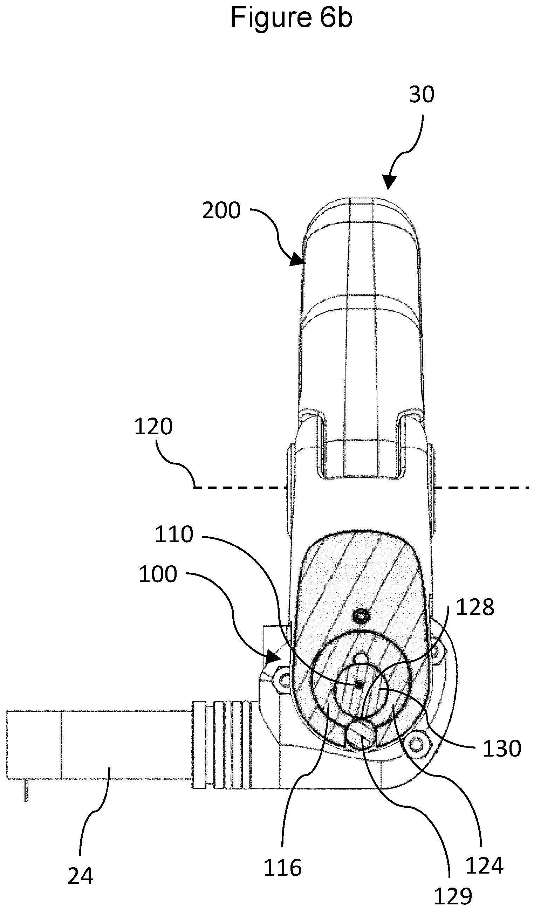

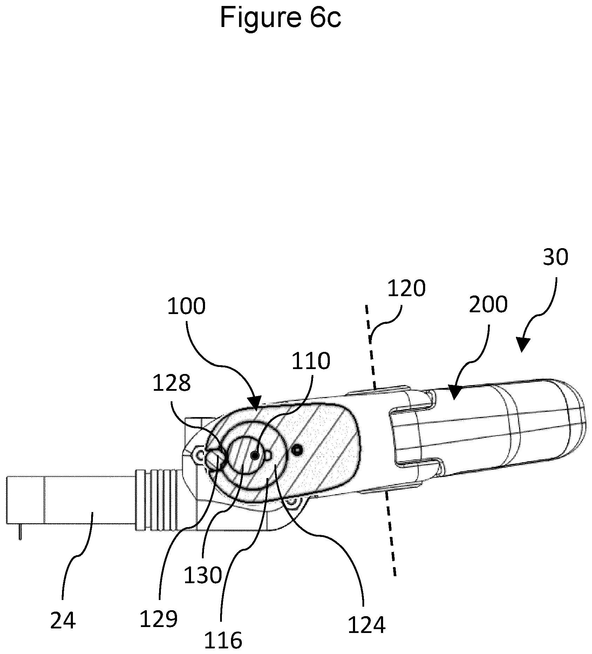

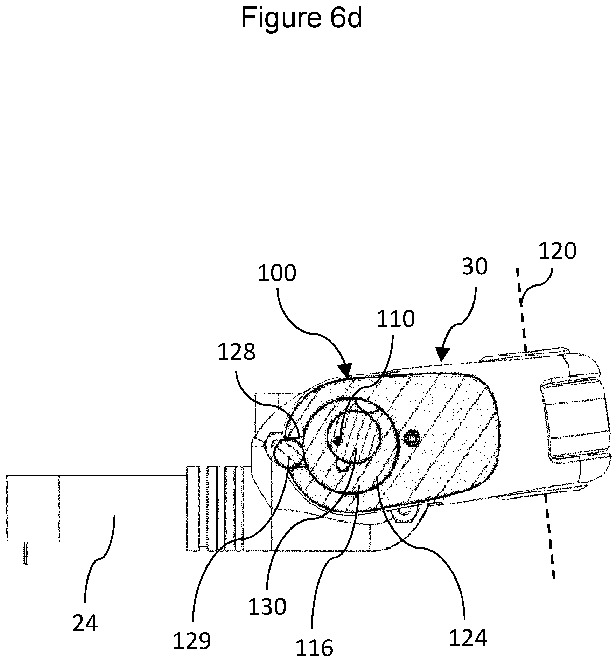

The prosthetic hand 10 may comprise a locking mechanism. The locking mechanism may comprise a first configuration and a second configuration. In the first configuration, the distal thumb part 200 may be rotatably locked relative to the proximal thumb part 100 about the second axis of rotation 120, such that the rotary actuator 24 rotates both the distal thumb part 200 and proximal thumb part 100 about the first axis of rotation 110. In the second configuration, the proximal thumb part 100 may be rotatably locked relative to the palm part 20 about the first axis of rotation 110 such that the rotary actuator 24 rotates the distal thumb part 200 relative to the proximal thumb part 100 about the second axis of rotation 120. In one example, e.g. as shown in Figure 6, the shaft 116 may comprise at least a part of the locking mechanism. In particular, the lock portion 124 of the shaft 116 may comprise the locking mechanism.

Referring to Figure 6a, the lock portion 124 part or portion of the shaft 116 may comprise at least one recess 128. The at least one recess 128 of the shaft lock portion 124 may be configured to receive at least a portion of locking element 129. The locking element 129 may be disposed adjacent the shaft lock portion 124. The locking element 129 may be disposed between the shaft lock portion 124 and the palm part 20. The locking element 129 may be disposed between the shaft lock portion 124 and a surrounding casing of the proximal thumb part 100. Similarly, and referring now to Figure 9, the palm part 20 may comprise at least one recess 27. For example, the palm part 20 may comprise a first recess 27a and a second recess 27b. The at least one recess 27 of the palm part 20 may be disposed adjacent or proximate to the proximal thumb part 100. Specifically, the at least one recess 27 of the palm part 20 may be disposed proximate or adjacent to the shaft lock portion 124 of the proximal thumb part 100. The at least one recess 27 of the palm part 20 may be configured to receive at least a portion of the locking element 129. Accordingly, in at least some configurations, the at least one recess 27 of the palm part 20 may be disposed proximate or adjacent to the locking element 129. The locking element 129 may be moveable between a first position and a second position. **In** the first position, the locking element 129 may disengage or move out of the recess 128 of the shaft lock portion 124. The locking element 129 may disengage or move out of the recess 128 of the shaft lock portion 124 and move into or engage one of the at least one recesses 27 of the palm part 20. **In** particular, the locking element 129 may move radially outward from the recess 128 of the shaft lock portion 124 and be received by recess 27a or recess 27b of the palm part 20. Therefore, in a first position, the locking element 129 may engage either recess 27a or recess 27b of the palm part 20. In the second position, the locking element 129 may engage or move into the recess 128 of the shaft lock portion 124. For example, the locking element 129 may move radially inward from recess 27a or recess 27b of the palm part 20 and be received by the recess 128 of the shaft lock portion 124. The locking element 129 may be a spherical ball. The locking element 129 may alternatively be of a different shape. At least a portion of the locking element 129 may be magnetic. The shaft lock portion 124 may further comprise a biasing element 130. The biasing element 130 may be configured to bias the locking element 129 into the second position. **In** other words, the biasing element 130 may be configured to bias the locking element 129 into the recess 128 of the shaft lock portion 124. **In** one example, if the locking element 129 is at least partially magnetic, the biasing element 130 may also be at least partially magnetic. Accordingly, the locking element 129 and the biasing element 130 may be magnetically attracted to one another. Therefore, the locking element 129 may be pulled or attracted into recess 128 of the shaft lock portion 124 by the biasing element 130.

When the locking element 129 is in the first position, e.g. as shown in Figures 6a and 6d, the shaft lock portion 124 may be rotatable relative to the proximal thumb part 100 about the first axis of rotation 110. When the locking element 129 is in the second position, e.g. as shown in Figures 6b and 6c, the shaft lock portion 124 may be rotatably locked relative to the proximal thumb part 100 about the first axis of rotation 110. The locking element 129, in engaging the recess 128 of the lock portion 124 of the shaft 116, prevents relative movement between the shaft 116 and the surrounding casing of the proximal thumb part 100. Similarly, the locking element 129, in disengaging the recess 128 of the lock portion 124 of the shaft 116, enables the shaft 116 to rotate relative to a surrounding casing of the proximal thumb part 100.

Accordingly, as shown in Figure 6a, when the thumb part 30 is in a closed opposition position, the locking element 129 may be in the first position (i.e., the locking element 129 may not engage the recess 128 of the lock portion 124 of the shaft 116). Since the locking element 129 is in the first position, a torque transmitted from the rotary actuator 24 may cause the shaft 116 to rotate clockwise about the first axis of rotation 110 relative to a surrounding casing of the proximal thumb part 100. Such rotation of the shaft 116 may cause the arm 220 (see Figure 5) to exert a force on the distal thumb part 200, such that the distal thumb 200 rotates about the second axis of rotation 120 from the closed position to the open position. The shaft 116 may rotate clockwise until the recess 128 is proximate or adjacent the locking element 129. When the recess 128 is adjacent the locking element 129, as shown in Figure 6b, the biasing element 130 may pull, attract, or otherwise force the locking element 129 into the recess 128. Since the locking element 129 is now in the second position, torque transmitted from the rotary actuator 24 may be transmitted into the surrounding casing of the proximal thumb part 100, to thereby cause the thumb part 30 (i.e. the proximal thumb part 100 and the distal thumb part 200) to rotate clockwise about the first axis of rotation 110 from the open opposition position (shown in Figure 6b) to the open reposition position (shown in Figure 6c). Once the proximal thumb part 100 reaches a limit of its travel (either the opposition position or the reposition position), further torque transmitted from the rotary actuator 24 may urge the locking element 129 to move into the other of the first or second position. As shown in Figure 6c, since the proximal thumb part 100 has reached a limit of its travel (the reposition position), any further torque imparted by the rotary actuator 24 may urge or force the locking element 129 out of the recess 128. In other words, any further torque imparted by the rotary actuator 24 may urge or force the locking element 129 from the second position into the first position. Since the locking element 129 is now back in the first position, further torque transmitted from the rotary actuator 24 may cause the shaft 116 to rotate clockwise about the first axis of rotation 110 relative to a surrounding casing of the proximal thumb part 100, such that the arm 220 (see Figure 5) exerts a force on the distal thumb part 200. The distal thumb part 200 may consequently rotate about the second axis of rotation 120 from the open position to the closed position shown in Figure 6d. It is noted that the rotary actuator 24 may be actuated in both clockwise and anti-clockwise directions. Accordingly, in a similar manner as described in relation to moving the thumb part 30 from the closed opposition position to the closed reposition position, the shaft 116 may be driven anti-clockwise to thereby move the thumb part from the closed reposition shown in Figure 6d to the closed opposition position shown in Figure 6a.

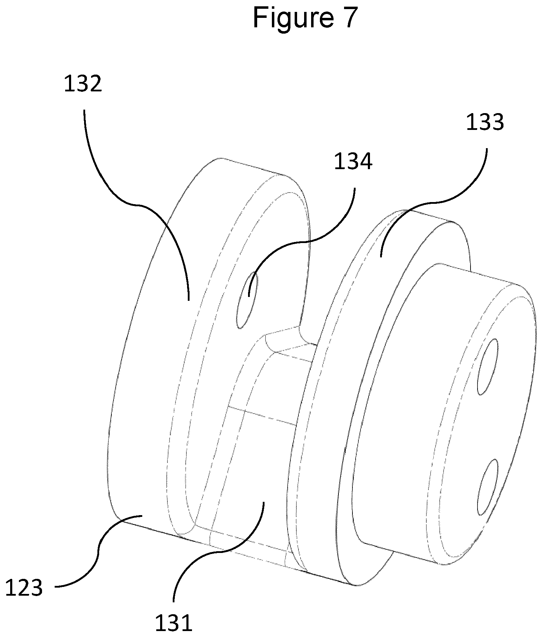

Turning now to Figure 7, the portion or part of the shaft 116 comprising the coupling mount 123 may comprise a first large diameter portion 132 and a second large diameter portion 133. The coupling mount 123 may comprise a cavity. The cavity may be disposed between the first and second large diameter portions 132, 133. Accordingly, the first large diameter portion 132 may be spaced apart from the second large diameter portion 133. An abutment portion 131 may be formed between the first large diameter portion 132 and the second large diameter portion 133. A length between the opposing faces of the first and second large diameter portions 132, 133 may be equal to or greater than a width of the arm 220 (shown in Figure 5 and Figure 8). Therefore, at least a portion of the arm 220, e.g. the first end 222 of the arm 220, may be received in the space formed between the large diameter portions 132 and 133 of the coupling mount 123. At least one of the first large diameter portion 132 and the second large diameter portion 133 may comprise a pinhole 134 configured to receive a hinge pin 135. The first end 222 of the arm 220 may thus be slotted between the large diameter portions 132, 133, and thereby coupled to the coupling mount 123. Specifically, the arm 220 may be coupled to the coupling mount 123 by way of a hinge pin 135 inserted through the pinhole 134 and through the first end 222 of the arm 220. The first end 222 of the arm 220 may thus be configured to be disposed over the abutment portion 131.

Referring now to Figure 8, since the arm 220 is coupled to the coupling mount 123 by hinge pin 135, rotation of the coupling mount 123 (as may occur when the locking element 129 is in the first position) may cause at least a part of the arm 220, e.g. the first end 222 of the arm 220, to rotate about the first axis of rotation 110. Torque may thus be transmitted along the arm 220 to the second end 224 of the arm 220. As the second end 224 of the arm 220 is pivotally coupled or hinged to the distal thumb part 200 by pin 226, the torque transmitted along the arm 220 may cause the distal thumb part 200 to rotate about the second axis of rotation 120 between the open and closed positions.

Still referring to Figure 8, the distal thumb part 200 may be prevented from over-rotating past the closed position by the abutment portion 131. In particular, the abutment portion 131 may be substantially wedge shaped. The abutment portion 131 may comprise two slanted surfaces which meet at a leading edge. Therefore, when the distal thumb part 200 is in the open position (as shown in Figure 8a), the first end 222 of the arm 220 may extend away from and/or be proximate to the leading edge of the abutment portion 131 of the coupling mount 123. On the other hand, when the distal thumb part 200 is rotated to the closed position (as shown in Figure 8b), a surface of the first end 222 of the arm 220 may abut or come into contact with one of the two slanted surfaces of the abutment portion 131, thereby preventing further rotation of the distal thumb part 200.

As shown in Figure 8, the arm 220 may comprise three links. A first link 228, a second link 230, and a third link 232. The first link 228 may be disposed proximate to the proximal thumb part 100. The first link 228 may comprise the first end 222 of the arm 220. Accordingly, at one end, the first link 228 may be pivotally coupled to the shaft 116 by first hinge pin 135. At the other end, the first link 228 may be pivotally coupled to the second link 230. The second link 230 may couple the first link 228 to the third link 232. The second link 230 and the first link 228 may be pivotally coupled, e.g. by a second hinge pin. Similarly, the second link 230 and the third link 232 may be pivotally coupled, e.g. by a third hinge pin. The third link 232 may comprise the second end 224 of the arm 220. Accordingly, the third link 232 may be disposed proximate to the distal thumb part 200. The third link 232 may be pivotally coupled or hinged to the distal thumb part 200 by fourth hinge pin 226. A longitudinal axis of the first hinge pin 135 may be substantially parallel to the first axis of rotation 110. A longitudinal axis of the fourth hinge pin 226 may be substantially parallel to the second axis of rotation 120. Accordingly, the second link 230 may be coupled to the first link 228 such that a longitudinal axis of the second hinge pin (i.e. the pin coupling the first link 228 to the second link 230) is substantially parallel to the longitudinal axis of the first hinge pin 135 and/or substantially parallel to the first axis of rotation 110. Similarly, the second link 230 may be coupled to the third link 232 such that a longitudinal axis of the third hinge pin (i.e. the pin coupling the second link 230 to the third link 232) is substantially parallel to the longitudinal axis of the fourth hinge pin 226 and/or substantially parallel to the second axis of rotation 120. Accordingly, rotation of the first link 228 about the first axis of rotation 110 may result in the third link 232 exerting a force on the distal thumb part 200 that urges the distal thumb part 200 to rotate about the second axis of rotation 120.

Rotation of the thumb part 30 may be described by way of a sequence of events. For example, rotation of the thumb part 30 from the position shown in Figure 1a, i.e., with the proximal thumb part 100 in the reposition position and the distal thumb part 200 in the open position, may be described by way of the following sequence of events:
1. The actuator 24 is caused (e.g. by a user) to drive the gear mechanism 111 (e.g. worm screw 112 and worm gear 114), which in turn drives the shaft 116.
2. Anticlockwise rotation of shaft 116 (as viewed in Fig. 9) pushes locking element 129 radially outward and into the first palm recess 27a. Accordingly, the shaft 116 is now able to rotate relative to the proximal thumb part 100, and the proximal thumb part 100 is fixed relative to the palm part 20.
3. Further anticlockwise rotation of the shaft 116 causes the arm 220 (e.g. first link 228 of the arm 220) to move, such that the distal thumb part 200 rotates relative to the proximal thumb part 100.
4. Rotation of the shaft 116 continues until the first end 222 of the arm 220 (e.g. first link 228 of the arm 220) abuts the abutment portion 131, at which point the distal thumb part 200 is in the closed position (e.g. as shown in Fig 2b).
5. Rotation of actuator 24 can then be reversed.
6. Relative rotation of the proximal thumb part 100 and the palm part 20 is still prevented by the locking element 129, which is disposed in the first palm recess 27a. The shaft 116 therefore rotates in a clockwise direction (as viewed in Fig. 9), which causes the distal thumb part 200 to move back into the open position.
7. When the distal thumb part 200 is in the open position, the locking interface recess 128 may align with the locking element 129, such that the biasing element 130 (e.g. the magnet) urges the locking element 129 back towards the shaft 116 (and away from the first palm part recess 27a). The shaft 116 and the proximal thumb part 100 are then rotatably locked together, but the proximal thumb part 100 and the palm part 20 are free to rotate.
8. Further clockwise rotation of the shaft 116 causes the proximal thumb part 100 to rotate relative to the palm part 20. The thumb then moves into the opposition position (e.g. as shown in Fig 1b).
9. Further clockwise rotation of the shaft pushes the locking element 129 radially outward and into the second palm recess 27b. Accordingly, the shaft 116 is now able to rotate relative to the proximal thumb part 100, and the proximal thumb part 100 is fixed relative to the palm part 20.
10. Further clockwise rotation of the shaft 116 causes the arm 220 (e.g. first link 228 of the arm 220) to move, such that the distal thumb part 200 rotates relative to the proximal thumb part 100.
11. Rotation of the shaft 116 continues until the first end 222 of the arm 220 (e.g. first link 228 of the arm 220) abuts the abutment portion 131, at which point the distal thumb part 200 is in the closed position.
12. Rotation of the actuator 24 can then be reversed.
13. Relative rotation of the proximal thumb part 100 and the palm part 20 is still prevented by the locking element 129, which is disposed in the second palm recess 27b. Anticlockwise rotation of the shaft 116 causes the distal thumb part 200 to move back into the open position.
14. When the distal thumb part 200 is in the open position, the locking interface recess 128 may align with the locking element 129, such that the biasing element 130 (e.g. the magnet) urges the locking element 129 back towards the shaft 116 (and away from the second palm part recess 27b). The shaft 116 and the proximal thumb part 100 are then rotatably locked together, but the proximal thumb part 100 and the palm part 20 are free to rotate.
15. Further anticlockwise rotation of the shaft 116 causes the proximal thumb part 100 to rotate relative to the palm part 20. The thumb part 30 then moves back into the reposition position (e.g. as shown in Fig 1a).
16. The cycle may then be repeated.

Turning back to Figure 5, the hinge pin 226 may slot into or be disposed in a groove 234 formed on the distal thumb part 200. Pin 226 may extend from groove 234, through the second end 224 of the arm 220, and into a corresponding groove formed on the other side of the distal thumb part 200. Although not shown, the distal thumb part 200 may further comprise a biasing spring, such as a torsion spring. The biasing spring may be configured to return the distal thumb part 200 to a neutral position. The neutral position of the distal thumb part 200 may be when the pin 226 is at an end of the groove 234. The biasing spring may thus be configured to return the pin 226 to the end of the groove 234. In other words, in the absence of an external force, the pin 226 may be disposed at the end of the groove 234, such that the distal thumb part 200 is in its neutral position. For example, if the distal thumb part 200 is pushed against an object, the pin 226 may move along groove 234 to thereby permit the distal thumb part 200 to flex about the second axis of rotation 120. In the absence of the external force, e.g. if the distal thumb part is no longer pressed up against the object, the pin 226 may return, snap, or otherwise spring back to its original position at the end of the groove 234.

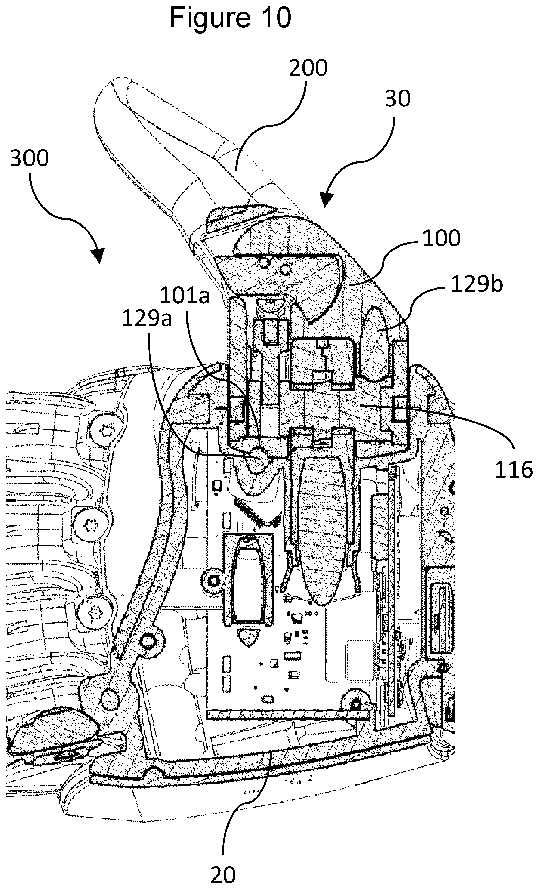

Figures 10 to 13 depict a prosthetic hand 300 according to a further example of the present disclosure. The prosthetic hand 300 is similar to the prosthetic hand 10 in that it comprises palm part 20 and thumb part 30, which in turn comprises proximal thumb part 100 and distal thumb part 200. Features relating to these components are the same as those described above and will not be repeated here. The following description focusses on the differences between the two examples.

As for the prosthetic hand 10, the prosthetic hand 300 may comprise a locking mechanism. The locking mechanism of the prosthetic hand 300 may comprise a first configuration and a second configuration. In the first configuration, the distal thumb part 200 may be rotatably locked (e.g. restricted) relative to the proximal thumb part 100 about the second axis of rotation 120, such that the rotary actuator 24 rotates both the distal thumb part 200 and proximal thumb part 100 about the first axis of rotation 110. In the second configuration, the proximal thumb part 100 may be rotatably locked (e.g. restricted) relative to the palm part 20 about the first axis of rotation 110 such that the rotary actuator 24 rotates the distal thumb part 200 relative to the proximal thumb part 100 about the second axis of rotation 120. However, the prosthetic hand 300 according to the further example of the present disclosure differs in that the locking mechanism comprises first and second movable locking elements 129a, 129b.

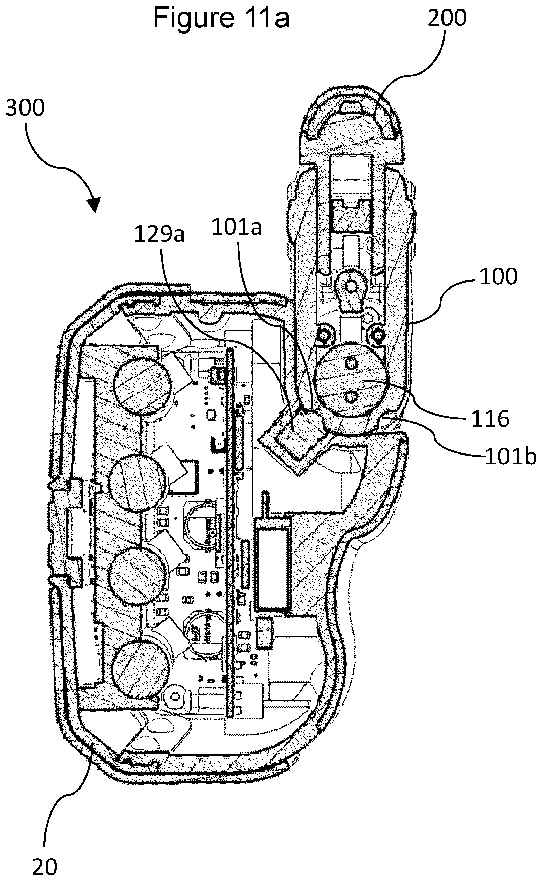

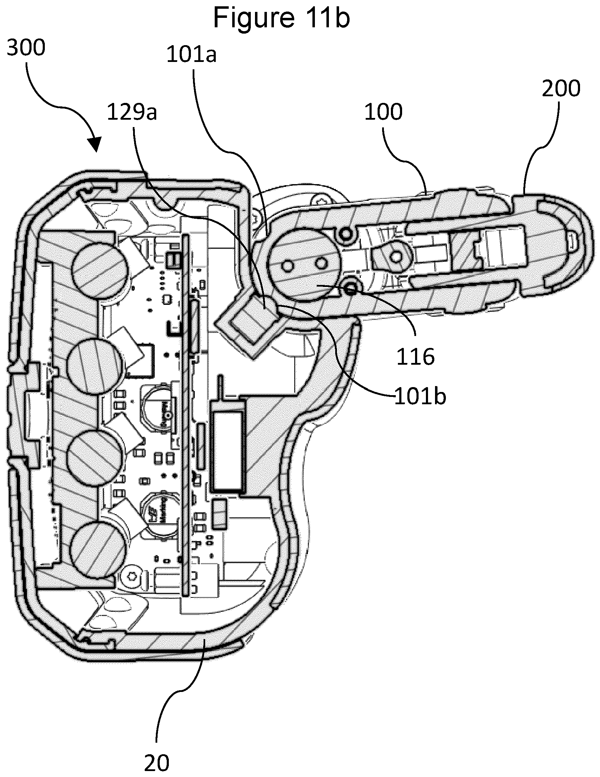

As shown in Figures 10 and 11, the first movable locking element 129a may be configured to lock (e.g. restrict) relative movement of the palm part 20 and proximal thumb part 100 when the first movable locking element 129a is in an extended position. The first movable locking element 129a may extend from the palm part 20 and may selectively extend into one or more recesses 101 in the proximal thumb part 100 when in the extended position. Figure 11a shows the first movable locking element 129a extending into a first recess 101a and Figure 11b shows the first movable locking element 129a extending into a second recess 101b. The first and second recesses 101a, 101b may correspond to the limits of the travel for the proximal thumb part 100.

The first movable locking element 129a may be resiliently biased into the extended position, e.g. by virtue of a spring (not shown). The first movable locking element 129a may form part of a first spring-loaded plunger. The first spring-loaded plunger may be provided in the palm part 20.

The first movable locking element 129a may be configured to permit relative movement of the palm part 20 and proximal thumb part 100 when the first movable locking element 129a is in a retracted position, e.g. when the first movable locking element 129a does not engage the recesses 101 in the proximal thumb part 100. The first movable locking element 129a may slide against a surface of the proximal thumb part 100 between the recesses 101a, 101b.

The first movable locking element 129a and/or recesses 101 may be curved. Such curvature may cause the first movable locking element 129a to be retracted when a sufficient force is applied to the proximal thumb part 100 (e.g. by virtue of the rotation of the first shaft 116 by the rotary actuator 24) to overcome the resilient biasing force acting on the first movable locking element 129a.

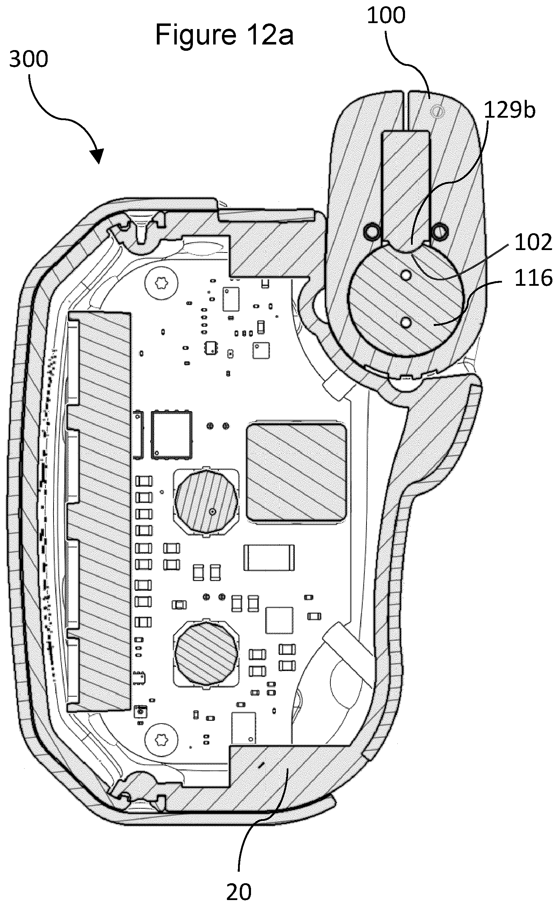

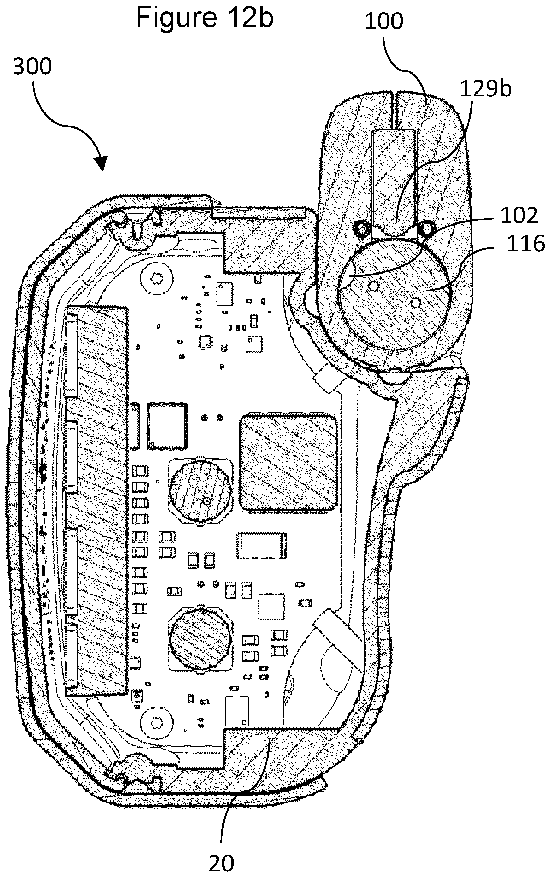

As shown in Figures 10 and 12, the second movable locking element 129b may be configured to lock (e.g. restrict) relative movement of the distal thumb part 200 and proximal thumb part 100 when the second movable locking element 129b is in an extended position. The second movable locking element 129b may extend from the proximal thumb part 100 and may selectively extend into a recess 102 in the first shaft 116 when in the extended position (thereby restricting rotation of the shaft 116 relative to the proximal thumb part 100 and thus restricting movement of the distal thumb part 200 relative to the proximal thumb part 100). Figure 12a shows the first movable locking element 129a extending into the recess 102 and Figure 12b shows the first movable locking element 129a retracted from the recess 102. The second movable locking element 129b may extend into the recess 102 when the distal thumb part 200 is in the open position.

The second movable locking element 129b may be resiliently biased into the extended position, e.g. by virtue of a spring (not shown). The second movable locking element 129b may form part of a second spring-loaded plunger. The second spring-loaded plunger may be provided in the proximal thumb part 100.

The second movable locking element 129b may be configured to permit relative movement of the distal thumb part 200 and proximal thumb part 100 when the second movable locking element 129b is in a retracted position, e.g. when the second movable locking element 129b does not engage the recess 102 in the first shaft 116. The second movable locking element 129b may slide against a surface of the shaft 116 when not in the extended position.

The second movable locking element 129b and/or recess 102 may be curved. Such curvature may cause the second movable locking element 129b to be retracted when a sufficient force is applied to the distal thumb part 200 (e.g. by virtue of the rotation of the first shaft 116 by the rotary actuator 24) to overcome the resilient biasing force acting on the second movable locking element 129b.

The force required to overcome the resilient biasing force acting on the second movable locking element 129b may be greater than the force required to overcome the resilient biasing force acting on the first movable locking element 129a. This difference may be achieved by setting the resilient biasing forces (e.g. of the plunger springs) of the first and second movable locking elements 129a, 129b as required. In this way, rotation of the first shaft 116 may preferentially move the proximal thumb part 100 relative to the palm part 20 before moving the distal thumb part 200 away from its open position.

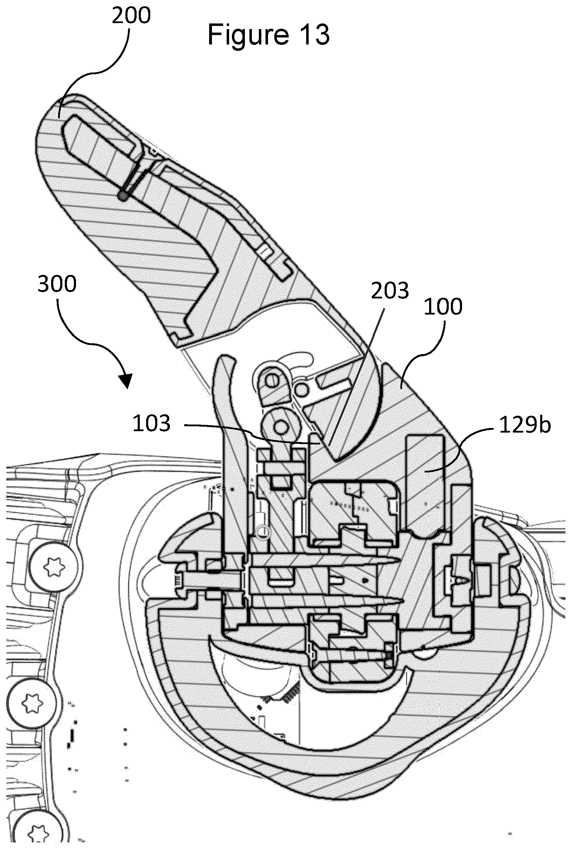

With reference to Figure 13, the proximal thumb part 100 may comprise an abutment surface 103 that may engage a corresponding abutment surface 203 on the distal thumb part 200, e.g. when the distal thumb part 200 is in the open position relative to the proximal thumb part 100.

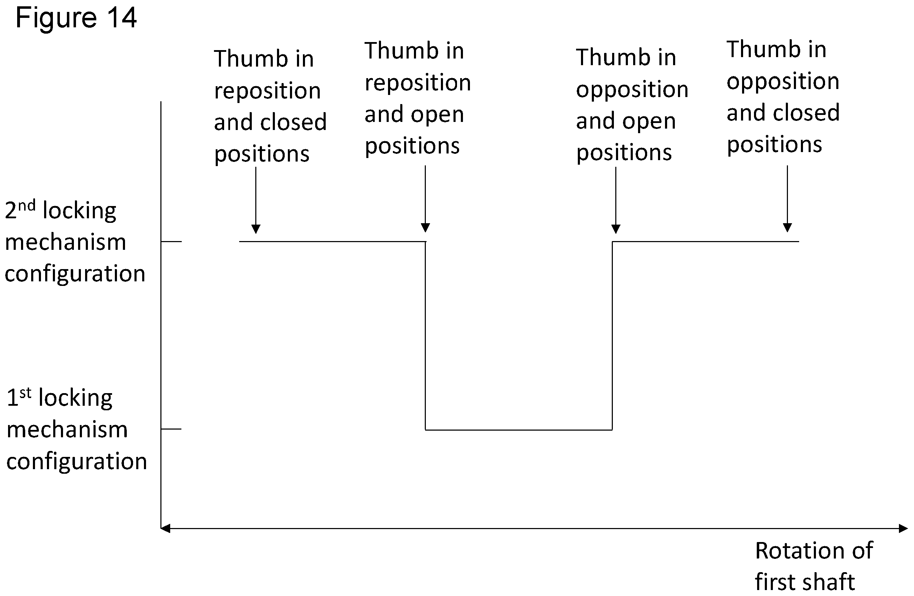

Figure 14 depicts the progression of the thumb part 30 through its different positions for either of the prosthetic hands 10, 300. More specifically, for the prosthetic hand 300, as the first shaft 116 rotates, the thumb may cycle through the positions in the following order:
(i) A first setting in which the distal thumb part 200 is in a closed position and the thumb part is in a reposition position. The first movable locking element 129a extends into first recess 101a and the second movable locking element 129b is retracted.
(ii) A second setting in which the distal thumb part 200 is in an open position and the thumb part is in a reposition position. The first movable locking element 129a extends into first recess 101a and the second movable locking element 129b extends into recess 102.
(iii) A third setting in which the distal thumb part 200 is in an open position and the thumb part is in an opposition position. The first movable locking element 129a extends into second recess 101b and the second movable locking element 129b extends into recess 102.
(iv) A fourth setting in which the distal thumb part 200 is in a closed position and the thumb part is in an opposition position. The first movable locking element 129a extends into second recess 101b and the second movable locking element 129b is retracted.

Rotation of the first shaft 116 may then be reversed so that the thumb may cycle through the settings in the reverse order.

## Claims

1. A prosthetic hand comprising:
a palm part (20);
a proximal thumb part (100) coupled to the palm part to form a first joint with a first axis of rotation (110),
wherein the proximal thumb part is configured to rotate about the first axis of rotation;
a distal thumb part (200) coupled to the proximal thumb part to form a second joint with a second axis of rotation (120),
wherein the distal thumb part is configured to rotate about the second axis of rotation; **characterised by** a rotary actuator (24) configured to rotate the proximal thumb part and distal thumb part about the first axis of rotation and the second axis of rotation respectively; and
a locking mechanism having a first configuration and a second configuration, the locking mechanism being configured such that:
in the first configuration, the distal thumb part is rotatably locked relative to the proximal thumb part about the second axis of rotation such that the rotary actuator rotates both the distal thumb part and proximal thumb part about the first axis of rotation, and
in the second configuration, the proximal thumb part is rotatably locked relative to the palm part about the first axis of rotation such that the rotary actuator rotates the distal thumb part relative to the proximal thumb part about the second axis of rotation.

2. The prosthetic hand of claim 1, wherein the proximal thumb part rotates between first and second limits of travel and the locking mechanism is configured to switch between the first and second configurations at at least one of the first and second limits of travel.

3. The prosthetic hand of claim 1 or 2, wherein the locking mechanism comprises a movable locking element (129), the movable locking element being configured to prevent relative movement of the palm part and proximal thumb part when in a first position and prevent relative movement of the distal thumb part and proximal thumb part when in a second position.

4. The prosthetic hand of claim 2 and 3, wherein the locking mechanism is configured such that the locking element is urged into the first position by movement of the distal thumb part when the proximal thumb part is at the first and/or second limits of travel.

5. The prosthetic hand of claim 3 or 4, wherein the locking mechanism comprises a biasing element (130) configured to bias the locking element into the second position.

6. The prosthetic hand of claim 5, wherein the biasing element and the locking element are magnetically attracted to one another.

7. The prosthetic hand of claim 1 or 2, wherein the locking mechanism comprises a first movable locking element (129a) configured to restrict relative movement of the palm part and proximal thumb part when the first movable locking element is in an extended position, and a second movable locking element (129b) configured to restrict relative movement of the distal thumb part and proximal thumb part when the second movable locking element is in an extended position.

8. The prosthetic hand of claim 7, wherein the first movable locking element is part of a spring-loaded plunger and/or the second movable locking element is part of a spring-loaded plunger.

9. The prosthetic hand of any preceding claim, wherein the proximal thumb part comprises a first shaft (116) configured to rotate about the first axis of rotation, the first shaft being positioned such that the first axis of rotation passes through the first shaft, e.g. wherein a longitudinal axis of the first shaft is substantially parallel and coincidental to the first axis of rotation.

10. The prosthetic hand of claim 9, wherein the first shaft comprises a recess (128), and the locking mechanism comprises a locking element configured to engage the recess, and wherein rotation of the first shaft relative to a surrounding casing (119) of the proximal thumb part is restricted when the locking element engages the recess, and the first shaft is rotatable relative to the surrounding casing of the proximal thumb part when the locking element does not engage the recess.

11. The prosthetic hand of claim 10, wherein the locking element comprises an at least partially magnetic object, and the first shaft comprises a magnet configured to attract the at least partially magnetic object into the recess.

12. The prosthetic hand of claim 10, wherein the locking element comprises a spring-loaded plunger.

13. The prosthetic hand of any of claims 9 to 12, wherein the palm part comprises the rotary actuator, and wherein the rotary actuator is configured to drive the first shaft through a gear mechanism (111).

14. The prosthetic hand of any preceding claim, wherein the second axis of rotation is substantially perpendicular to the first axis of rotation.

15. The prosthetic hand of any preceding claim, wherein the distal thumb part comprises a biasing spring, the biasing spring being configured to return the distal thumb part to a neutral position.

## Patentansprüche

1. Prothetische Hand, umfassend:
ein Handflächenteil (20);
ein proximales Daumenteil (100), das mit dem Handflächenteil gekoppelt ist, um ein erstes Gelenk mit einer ersten Drehachse (110) zu bilden,
wobei das proximale Daumenteil dazu ausgebildet ist, sich um die erste Drehachse zu drehen;
ein distales Daumenteil (200), das mit dem proximalen Daumenteil gekoppelt ist, um ein zweites Gelenk mit einer zweiten Drehachse (120) zu bilden,
wobei das distale Daumenteil dazu ausgebildet ist, sich um die zweite Drehachse zu drehen;
**gekennzeichnet durch** einen Drehantrieb (24), der dazu ausgebildet ist, das proximale Daumenteil und das distale Daumenteil um die erste Drehachse bzw. die zweite Drehachse zu drehen; und
einen Verriegelungsmechanismus mit einer ersten Konfiguration und einer zweiten Konfiguration, wobei der Verriegelungsmechanismus derart ausgebildet ist, dass:
in der ersten Konfiguration das distale Daumenteil relativ zu dem proximalen Daumenteil um die zweite Drehachse drehfest verriegelt ist, sodass der Drehantrieb sowohl das distale Daumenteil als auch das proximale Daumenteil um die erste Drehachse dreht, und
in der zweiten Konfiguration das proximale Daumenteil relativ zu dem Handflächenteil um die erste Drehachse drehfest verriegelt ist, sodass der Drehantrieb das distale Daumenteil relativ zu dem proximalen Daumenteil um die zweite Drehachse dreht.

2. Prothetische Hand nach Anspruch 1, wobei sich das proximale Daumenteil zwischen einer ersten und einer zweiten Bewegungsendlage dreht und der Verriegelungsmechanismus dazu ausgebildet ist, an mindestens einer der ersten und zweiten Bewegungsendlage zwischen der ersten und der zweiten Konfiguration umzuschalten.

3. Prothetische Hand nach Anspruch 1 oder 2, wobei der Verriegelungsmechanismus ein bewegliches Verriegelungselement (129) umfasst, wobei das bewegliche Verriegelungselement dazu ausgebildet ist, in einer ersten Position eine Relativbewegung des Handflächenteils und des proximalen Daumenteils zu verhindern und in einer zweiten Position eine Relativbewegung des distalen Daumenteils und des proximalen Daumenteils zu verhindern.

4. Prothetische Hand nach Anspruch 2 und 3, wobei der Verriegelungsmechanismus derart ausgebildet ist, dass das Verriegelungselement durch eine Bewegung des distalen Daumenteils in die erste Position gedrängt wird, wenn sich das proximale Daumenteil an der ersten und/oder zweiten Bewegungsendlage befindet.

5. Prothetische Hand nach Anspruch 3 oder 4, wobei der Verriegelungsmechanismus ein Vorspannelement (130) umfasst, das dazu ausgebildet ist, das Verriegelungselement in die zweite Position vorzuspannen.

6. Prothetische Hand nach Anspruch 5, wobei das Vorspannelement und das Verriegelungselement einander magnetisch anziehen.

7. Prothetische Hand nach Anspruch 1 oder 2, wobei der Verriegelungsmechanismus ein erstes bewegliches Verriegelungselement (129a), das dazu ausgebildet ist, eine Relativbewegung des Handflächenteils und des proximalen Daumenteils einzuschränken, wenn sich das erste bewegliche Verriegelungselement in einer ausgefahrenen Position befindet, und ein zweites bewegliches Verriegelungselement (129b) umfasst, das dazu ausgebildet ist, eine Relativbewegung des distalen Daumenteils und des proximalen Daumenteils einzuschränken, wenn sich das zweite bewegliche Verriegelungselement in einer ausgefahrenen Position befindet.

8. Prothetische Hand nach Anspruch 7, wobei das erste bewegliche Verriegelungselement Teil eines federbelasteten Stößels ist und/oder das zweite bewegliche Verriegelungselement Teil eines federbelasteten Stößels ist.

9. Prothetische Hand nach einem vorhergehenden Anspruch, wobei das proximale Daumenteil eine erste Welle (116) umfasst, die dazu ausgebildet ist, sich um die erste Drehachse zu drehen, wobei die erste Welle derart positioniert ist, dass die erste Drehachse durch die erste Welle verläuft, z. B. wobei eine Längsachse der ersten Welle im Wesentlichen parallel und deckungsgleich zu der ersten Drehachse ist.

10. Prothetische Hand nach Anspruch 9, wobei die erste Welle eine Aussparung (128) umfasst und der Verriegelungsmechanismus ein Verriegelungselement umfasst, das dazu ausgebildet ist, in die Aussparung einzugreifen, und wobei eine Drehung der ersten Welle relativ zu einem umgebenden Gehäuse (119) des proximalen Daumenteils eingeschränkt ist, wenn das Verriegelungselement in die Aussparung eingreift, und die erste Welle relativ zu dem umgebenden Gehäuse des proximalen Daumenteils drehbar ist, wenn das Verriegelungselement nicht in die Aussparung eingreift.

11. Prothetische Hand nach Anspruch 10, wobei das Verriegelungselement ein zumindest teilweise magnetisches Objekt umfasst und die erste Welle einen Magneten umfasst, der dazu ausgebildet ist, das zumindest teilweise magnetische Objekt in die Aussparung zu ziehen.

12. Prothetische Hand nach Anspruch 10, wobei das Verriegelungselement einen federbelasteten Stößel umfasst.

13. Prothetische Hand nach einem der Ansprüche 9-12, wobei das Handflächenteil den Drehantrieb umfasst und wobei der Drehantrieb dazu ausgebildet ist, die erste Welle über einen Getriebemechanismus (111) anzutreiben.

14. Prothetische Hand nach einem vorhergehenden Anspruch, wobei die zweite Drehachse im Wesentlichen senkrecht zu der ersten Drehachse ist.

15. Prothetische Hand nach einem vorhergehenden Anspruch, wobei das distale Daumenteil eine Vorspannfeder umfasst, wobei die Vorspannfeder dazu ausgebildet ist, das distale Daumenteil in eine neutrale Position zurückzuführen.

## Revendications

1. Prothèse de main comprenant :
une partie de paume (20) ;
une partie proximale du pouce (100) couplée à la partie de paume pour former une première articulation avec un premier axe de rotation (110),
dans laquelle la partie proximale du pouce est configurée pour tourner autour du premier axe de rotation ;
une partie distale du pouce (200) couplée à la partie proximale du pouce pour former une seconde articulation avec un second axe de rotation (120),
dans laquelle la partie distale du pouce est configurée pour tourner autour du second axe de rotation ;
**caractérisée par** un actionneur rotatif (24) configuré pour faire tourner la partie proximale du pouce et la partie distale du pouce autour du premier axe de rotation et du second axe de rotation, respectivement ; et
un mécanisme de verrouillage ayant une première configuration et une seconde configuration, le mécanisme de verrouillage étant configuré de sorte que :
dans la première configuration, la partie distale du pouce est verrouillée en rotation par rapport à la partie proximale du pouce autour du second axe de rotation de sorte que l'actionneur rotatif fait tourner à la fois la partie distale du pouce et la partie proximale du pouce autour du premier axe de rotation, et
dans la seconde configuration, la partie proximale du pouce est verrouillée en rotation par rapport à la partie de paume autour du premier axe de rotation de sorte que l'actionneur rotatif fait tourner la partie distale du pouce par rapport à la partie proximale du pouce autour du second axe de rotation.

2. Prothèse de main selon la revendication 1, dans laquelle la partie proximale du pouce tourne entre des première et seconde limites de déplacement et le mécanisme de verrouillage est configuré pour basculer entre les première et seconde configurations au niveau d'au moins l'une des première et seconde limites de déplacement.

3. Prothèse de main selon la revendication 1 ou 2, dans laquelle le mécanisme de verrouillage comprend un élément de verrouillage mobile (129), l'élément de verrouillage mobile étant configuré pour empêcher le mouvement relatif de la partie de paume et de la partie proximale du pouce lorsqu'il est dans une première position et pour empêcher le mouvement relatif de la partie distale du pouce et de la partie proximale du pouce lorsqu'il est dans une seconde position.

4. Prothèse de main selon les revendications 2 et 3, dans laquelle le mécanisme de verrouillage est configuré de sorte que l'élément de verrouillage est sollicité vers la première position par le mouvement de la partie distale du pouce lorsque la partie proximale du pouce se trouve au niveau de la première et/ou de la seconde limite de déplacement.

5. Prothèse de main selon la revendication 3 ou 4, dans laquelle le mécanisme de verrouillage comprend un élément de rappel (130) configuré pour rappeler l'élément de verrouillage dans la seconde position.

6. Prothèse de main selon la revendication 5, dans laquelle l'élément de rappel et l'élément de verrouillage sont attirés magnétiquement l'un vers l'autre.

7. Prothèse de main selon la revendication 1 ou 2, dans laquelle le mécanisme de verrouillage comprend un premier élément de verrouillage mobile (129a) configuré pour limiter le mouvement relatif de la partie de paume et de la partie proximale du pouce lorsque le premier élément de verrouillage mobile est dans une position étendue, et un second élément de verrouillage mobile (129b) configuré pour limiter le mouvement relatif de la partie distale du pouce et de la partie proximale du pouce lorsque le second élément de verrouillage mobile est dans une position étendue.

8. Prothèse de main selon la revendication 7, dans laquelle le premier élément de verrouillage mobile fait partie d'un piston à ressort et/ou le second élément de verrouillage mobile fait partie d'un piston à ressort.

9. Prothèse de main selon une quelconque revendication précédente, dans laquelle la partie proximale du pouce comprend un premier arbre (116) configuré pour tourner autour du premier axe de rotation, le premier arbre étant positionné de sorte que le premier axe de rotation passe à travers le premier arbre, par exemple dans laquelle un axe longitudinal du premier arbre est sensiblement parallèle et coïncident avec le premier axe de rotation.

10. Prothèse de main selon la revendication 9, dans laquelle le premier arbre comprend un évidement (128), et le mécanisme de verrouillage comprend un élément de verrouillage configuré pour venir en prise avec l'évidement, et dans laquelle la rotation du premier arbre par rapport à un boîtier environnant (119) de la partie proximale du pouce est limitée lorsque l'élément de verrouillage vient en prise avec l'évidement, et le premier arbre peut tourner par rapport au boîtier environnant de la partie proximale du pouce lorsque l'élément de verrouillage ne vient pas en prise avec l'évidement.

11. Prothèse de main selon la revendication 10, dans laquelle l'élément de verrouillage comprend un objet au moins partiellement magnétique, et le premier arbre comprend un aimant configuré pour attirer l'objet au moins partiellement magnétique dans l'évidement.

12. Prothèse de main selon la revendication 10, dans laquelle l'élément de verrouillage comprend un piston à ressort.

13. Prothèse de main selon l'une quelconque des revendications 9 à 12, dans laquelle la partie de paume comprend l'actionneur rotatif, et dans laquelle l'actionneur rotatif est configuré pour entraîner le premier arbre à travers un mécanisme d'engrenage (111).

14. Prothèse de main selon une quelconque revendication précédente, dans laquelle le second axe de rotation est sensiblement perpendiculaire au premier axe de rotation.

15. Prothèse de main selon une quelconque revendication précédente, dans laquelle la partie distale du pouce comprend un ressort de rappel, le ressort de rappel étant configuré pour ramener la partie distale du pouce à une position neutre.
